(19) Europäisches Patentamt / European Patent Office / Office européen des brevets

(11) EP 2 460 885 A1

(12) **EUROPEAN PATENT APPLICATION**
published in accordance with Art. 153(4) EPC

(43) Date of publication:
**06.06.2012 Bulletin 2012/23**

(21) Application number: **10804093.2**

(22) Date of filing: **26.07.2010**

(51) Int Cl.:
***C12P 41/00*** *(2006.01)*

(86) International application number:
**PCT/JP2010/004736**

(87) International publication number:
**WO 2011/013340 (03.02.2011 Gazette 2011/05)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.07.2009 JP 2009175660**

(83) **Declaration under Rule 32(1) EPC (expert solution)**

(71) Applicant: **Kaneka Corporation**
**Osaka-shi, Osaka 530-8288 (JP)**

(72) Inventors:
- **NOJIRI, Masutoshi**
  **Takasago-shi**
  **Hyogo 676-8688 (JP)**
- **UEKITA, Ken**
  **Takasago-shi**
  **Hyogo 676-8688 (JP)**
- **HIRAI, Yoshinori**
  **Takasago-shi**
  **Hyogo 676-8688 (JP)**
- **NISHIYAMA, Akira**
  **Takasago-shi**
  **Hyogo 676-8688 (JP)**
- **TAOKA, Naoaki**
  **Takasago-shi**
  **Hyogo 676-8688 (JP)**

(74) Representative: **HOFFMANN EITLE**
**Patent- und Rechtsanwälte**
**Arabellastraße 4**
**81925 München (DE)**

(54) **METHOD FOR PRODUCING OPTICALLY ACTIVE 3-SUBSTITUTED GLUTARIC ACID MONOAMIDE**

(57) The present invention relates to a method for producing optically active 3-substituted glutaric acid monoamide, which comprises allowing an enzyme source having activity of asymmetrically hydrolyzing a prochiral 3-substituted glutaric acid diamide compound to act on the compound. According to the present invention, a method for producing optically active 3-substituted glutaric acid monoamide, which is a useful drug intermediate, at a high yield from an inexpensive and readily available starting material.



Printed by Jouve, 75001 PARIS (FR)

## Description

### Technical Field

The present invention relates to a method for producing optically active 3-substituted glutaric acid monoamide, which is a useful drug intermediate.

### Background Art

The following methods are known examples of a method for producing optically active 3-substituted glutaric acid monoamide.

1) A method for obtaining optically active 3-substituted glutaric acid monoamide, by subjecting racemic 3-substituted glutaric acid monoamide to optical resolution via halogenation/crystallization or the like (Patent Literature 1 or Non-Patent Literature 1)
2) A method for obtaining optically active 3-substituted glutaric acid mono amide by obtaining optically active 3-substituted glutaric acid monoester via an asymmetric hydrolysis reaction of 3-substituted glutaric acid diester with the use of esterase or lipase and amidating the obtained optically active 3-substituted glutaric acid monoester (Non-Patent Literature 2).

However, method (1) is an optical resolution method. In such case, the yield cannot be expected to reach 50% or greater. Meanwhile, according to method (2), it is necessary to provide high pressure and low temperature conditions in the amidation step after asymmetric hydrolysis reaction.
As described above, it is difficult to regard conventionally known methods for producing optically active 3-substituted glutaric acid monoamide as industrially advantageous methods in consideration of yield and reaction conditions.

### Citation List

### Patent Literature

Patent Literature 1: WO97/225

Non-Patent Literature

Non-Patent Literature 1: Journal of the Chemical Society, Perkin Transactions 2: 1997(4), 763-768
Non-Patent Literature 2: Can. J. Chem., 72, 2312 (1994)

### Summary of Invention

### Technical Problem

In view of the above, an object of the present invention is to provide a method for producing optically active 3-substituted glutaric acid monoamide, which is a useful drug intermediate, at a high yield.

### Solution to Problem

As a result of intensive studies in order to achieve the above object, the present inventors succeeded in obtaining optically active 3-substituted glutaric acid monoamide at a high yield exceeding 50% under moderate pressure and temperature reaction conditions by obtaining a microoganism capable of asymmetrically hydrolyzing 3-substituted glutaric acid diamide in a stereoselective manner. This has led to the completion of the present invention.
Specifically, the present invention relates to a method for producing an optically active 3-substituted glutaric acid monoamide compound of the following formula (2), which comprises allowing an enzyme source having activity of asymmetrically hydrolyzing a 3-substituted glutaric acid diamide of the following formula (1) to act on the compound of (1):

Formula (1)

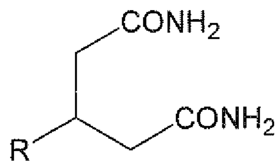

(1)

(where R represents a $C_1$-$C_8$ alkyl group that may optionally have a substituent, a $C_2$-$C_8$ alkenyl group that may optionally have a substituent, a $C_2$-$C_8$ alkynyl group that may optionally have a substituent, a $C_4$-$C_{20}$ aryl group that may optionally have a substituent, or a $C_5$-$C_{20}$ aralkyl group that may optionally have a substituent);

Formula (2)

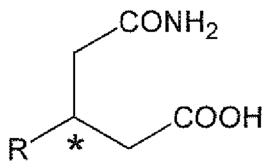

(2)

(where "*" represents an asymmetric carbon atom and R has the same definition as that above).

Advantageous Effects of Invention

According to the present invention, optically active 3-substituted glutaric acid monoamide can be produced at a high yield of 50% or greater with the use of prochiral 3-substituted glutaric acid diamide as a starting material.

Description of Embodiments

The present invention is described below in detail with reference to the embodiments. However, the present invention is not limited thereto.

The starting material of the present invention, 3-substituted glutaric acid diamide, is expressed by the following formula (1).

Formula (1)

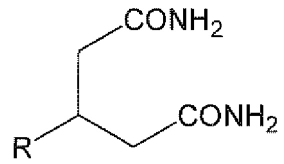

(1)

In formula (1), R represents a $C_1$-$C_8$ alkyl group that may optionally have a substituent, a $C_2$-$C_8$ alkenyl group that may optionally have a substituent, a $C_2$-$C_8$ alkynyl group that may optionally have a substituent, a $C_4$-$C_{20}$ aryl group that may optionally have a substituent, or a $C_5$-$C_{20}$ aralkyl group that may optionally have a substituent.

The $C_1$-$C_8$ alkyl group may be in a linear or branched form. Examples thereof include a methyl group, an ethyl group, an n-propyl group, an isopropyl group, an n-butyl group, an isobutyl group, a tert-butyl group, a pentanyl group, a hexanyl group, a heptanyl group, and an octanyl group.

Examples of the $C_2$-$C_8$ alkenyl group include an ethenyl group, a propenyl group, a butenyl group, a pentenyl group, a hexenyl group, a heptenyl group, and an octenyl group.

Examples of the $C_2$-$C_8$ alkynyl group include an ethinyl group, a propynyl group, a butynyl group, a pentynyl group, a hexynyl group, a heptynyl group, and an octynyl group.

Examples of the $C_4$-$C_{20}$ aryl group include a phenyl group, a naphthyl group, an anthranil group, a pyridyl group, a pyrimidyl group, an indanyl group, and an indenyl group.

Examples of the $C_5$-$C_{20}$ aralkyl group include a benzyl group, a naphthylmethyl group, an anthranilmethyl group, a pyridylmethyl group, a pyrimidylmethyl group, an indanylmethyl group, and an indenylmethyl group.

Each alkyl group, alkenyl group, alkynyl group, aryl group, and aralkyl group mentioned above may have a substituent. Examples of a substituent include a halogen atom, a hydroxyl group, an amino group, and a nitro group.

Specific examples of the aforementioned 3-substituted glutaric acid diamide of formula (1) include 3-propyl glutaric acid diamide, 3-isobutyl glutaric acid diamide, 3-phenyl glutaric acid diamide, and 3-(4-chlorophenyl) glutaric acid diamide.

Optically active 3-substituted glutaric acid monoamide obtained as a product of the present invention is expressed by the following formula (2).

Formula (2)

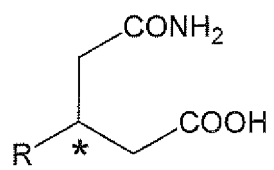

(2)

R in formula (2) has the same definition as that in formula (1).

For the compound of formula (2), the absolute configuration may be the R- or S-configuration. Specific examples of the compound include: 3-propyl glutaric acid monoamide, 3-isobutyl glutaric acid monoamide, 3-phenyl glutaric acid monoamide, and 3-(4-chlorophenyl) glutaric acid monoamide, for each of which the absolute configuration is the R-configuration; and 3-propyl glutaric acid monoamide, 3-isobutyl glutaric acid monoamide, 3-phenyl glutaric acid monoamide, and 3-(4-chlorophenyl) glutaric acid monoamide, for each of which the absolute configuration is the S-configuration.

The term "enzyme source" used in the present invention refers to microbial cells having activity of causing asymmetric hydrolysis of 3-substituted glutaric acid diamide, and a processed product of microbial cells.

The term "microbial cells" refers to not only cells of a microoganism but also a culture solution containing

microbial cells or a microbial cell suspension. In addition, examples of such microorganism include wild-type strains, mutant strains that have obtained advantageous properties via mutation of wild-type strains, and transformed strains transfected with DNA encoding the aforementioned enzyme from the microorganism that has activity of causing asymmetric hydrolysis.

The term "processed product of microbial cells" refers to, for example, a crude extract of microbial cells, lyophilized microbial cells, acetone-dried microbial cells, or a pulverized product of microbial cells. Further, the term may refer to an enzyme having activity of asymmetrically hydrolyzing 3-substitutcd glutaric acid diamide or a partially purified enzyme obtained therefrom. Moreover, a processed product of microbial cells can be used in the form of an immobilized enzyme or an immobilized microbial cell product obtained by immobilizing the enzyme or microbial cells. Immobilization can be performed by a method known to persons skilled in the art (e.g., a crosslinking method, a physical adsorption method, or an integrated immobilization method).

Microorganisms used herein, which are described below, are available at patent organism depositories and other research institutions, or they can be isolated from substances found in nature. For example, microorganisms specified by NBRC numbers are available at the NITE Biological Resource Center (NBRC) (NITE: the National Institute of Technology and Evaluation). Microorganisms specified by FERM numbers are available at the International Patent Organism Depository (IPOD), Advanced Industrial Science and Technology (AIST). Microorganisms specified by IAM numbers are available at the Center for Bioinfomatics, the Institute of Molecular and Cellular Biosciences, the University of Tokyo. Microorganisms specified by the JCM numbers are available at the Microbe Division, BioResource Center, RIKEN.

In addition, microorganisms can be isolated from substances found in nature via enrichment culture with the use of 3-substituted glutaric acid diamide, an inducer for inducing enzyme production, or the like.

Examples of an enzyme source having activity of asymmetrically hydrolyzing 3-substituted glutaric acid diamide include enzyme sources from microorganisms selected from the group consisting of members of the genera *Achromobacter, Alcaligenes, Arthrobacter, Acinetobacter, Cellulomonas, Comamonas, Delftia, Nocardia, Pseudomonas, Rhodococcus,* and *Stenotrophomonas.*

Among the above enzyme sources, examples of an enzyme source capable of producing 3-substituted glutaric acid monoamide, for which the absolute configuration is the S-configuration, include enzyme sources from microorganisms selected from the group consisting of members of the genera *Achrornobacter, Alcaligenes, Arthrobacter, Acinetobacter, Cellulomonas, Delftia, Pseudomonas,* and *Stenotrophomonas.*

Preferably, enzyme sources from the following are used: *Achromobacter* sp.; *Achromobacter xylosoxidans* subsp. *denitrificans; Alcaligerces xylosoxidans* subsp. *denitrificans; Arthrobacter* sp.; *Acinetobacter* sp.; *Cellulomonas fimi; Delftia acidovorans; Pseudomonas* sp.; and *Stenotrophomonas* sp.

More preferably, enzyme sources from the following are used: *Achromobacter xylosoxidans* subsp. *denitrificans* IFO15125; *Achromobacter xylosoxidans* subsp. *denitrificans* IFO12669; *Cellulomonas fimi* IFO15513; *Delftia* NBRC13582; and *Delftia* NBRC14950.

In addition, among the above enzyme sources, examples of an enzyme source capable of producing 3-substituted glutaric acid monoamide for which the absolute configuration is the R-configuration include enzyme sources from microorganisms selected from the group consisting of members of the genera *Alcaligenes, Acinetobacter, Comamonas, Nocardia, Rhodococcus,* and *Pseudomonas.*

Preferably, enzyme sources from *Alcaligenes* sp., *Acinetobacter* sp., *Comamonas* sp., *Nocardia globerula, Rhodococcus erythropolis, Rhodococcus rhodochrous,* and *Pseudomonas* sp. are used.

More preferably, enzyme sources from the following are used: *Alcaligenes* sp. IFO14130; *Comamonas* sp. KNK3-7 (NITE BP-963); *Rhodococcus erythropolis* IFO12538; *Rhodococcus erythropolis* TFQ12539; *Rhodococcus erythropolis* IAM1440; *Rhodococcus erythropolis* IAM1452; *Rhodococcus erythropolis* IAM1474; *Rhodococcus erythropolis* IAM12122; *Rhodococcus rhodochrous* NBRC15564; and *Pseudomonas* sp. KNK24-9 (NITE BP-962).

In addition, the aforementioned *Cornamonas* sp. KNK3-7 strain (NITE BP-963) and the *Pseudomonas* sp. KNK24-9 strain (NITE BP-962) were separated from soil by the present inventors and deposited as of July 7, 2010 with the above respective deposition numbers at the NITE Patent Microorganisms Depositary (NITE: the National Institute of Technology and Evaluation) (2-5-8 Kazusakamatari, Kisarazu-city, Chiba, Japan).

Further, a microorganism that can be used as the enzyme source of the present invention may be of a wild-type strain or a mutant strain. Alternatively, a microorganism obtained via a genetic means such as cell fusion or genetic engineering can be used.

For instance, a genetically engineered microoganism can be obtained by a method comprising: a step of determining either a partial or the entire amino acid sequence of an enzyme obtained via isolation and/or purification of an enzyme capable of asymmetrically hydrolyzing 3-substituted glutaric acid monoamide from any of the aforementioned microoganisms; a step of obtaining a DNA sequence encoding the enzyme based on such amino acid sequence; a step of obtaining a recombinant microoganism by introducing the obtained DNA into a different microoganism; and a step of obtaining the enzyme by culturing the recombinant microoganism. Each of the above steps can be carried out by a gene

recombination technique known to persons skilled in the art.

Examples of the above recombinant microoganism include transformed microorganisms obtained via transformation with the use of plasmids containing DNA encoding the above hydrolase. In addition, *Escherichia coli* is preferably used as a host microorganism.

A culture medium for a microorganism used as an enzyme source is not particularly limited as long as it allows the microoganism to proliferate. For example, a general liquid medium containing a carbon source (e.g., carbohydrate such as glucose or sucrose, alcohol such as ethanol or glycerol, fatty acid such as oleic acid or stearic acid or an ester thereof, or oil such as rapeseed oil or soybean oil), a nitrogen source (e.g., ammonium sulfate, sodium nitrate, peptone, casamino acid, corn steep liquor, bran, or yeast extract), an inorganic salt (e.g., magnesium sulfate, sodium chloride, calcium carbonate, potassium monohydrogen phosphate, or potassium dihydrogen phosphate), and an additional nutrient source (e.g., malt or meat extract) can be used.

In addition, an inducer may be added to a medium in order to induce enzyme production of a microorganism. Examples of an inducer include nitrile, a lactam compound, and amide. Examples of nitrile include acetonitrile, isovaleronitrile, propionitrile, pivalonitrile, n-butyronitrile, isobutyronitrile, n-capronitrile, and 3-penterlenitrile. Examples of a lactam compound include γ-butyrolactam, δ-valerolactam, and ε-caprolactam. Examples of amide include crotonamide, benzamide, propionamide, acetamide, n-butylamide, isobutylamide, n-valeramide, n-capronamide, methacrylamide, phenylacetamide, and cyclohexanecarboxamide.

The concentration of any of the above inducers in a medium is 0.05% to 2.0% and preferably 0.1% to 1.0%. Culture is carried out aerobically. In general, culture can be carried out under the following conditions: culture period: approximately 1 to 5 days; medium pH: 3 to 9; and culture temperature: 10°C to 50°C.

In the present invention, a stereoselective hydrolysis reaction of 3-substituted glutaric acid diamide (1) can be carried out by adding 3-substituted glutaric acid diamide (1) used as a starting material and the above microbial cells or a processed product thereof to an appropriate solvent, followed by stirring under controlled pH.

Reaction conditions would vary depending on the enzyme to be used, the microoganism or processed product thereof, the substrate concentration, and the like. However, in general, the substrate concentration is approximately 0.1% to 99% by weight and preferably 1% to 60% by weight. The reaction temperature is 10°C to 60°C and preferably 20°C to 50°C. The reaction pH is 4 to 11 and preferably 6 to 9. The reaction time is 1 to 120 hours and preferably 1 to 72 hours. A substrate can be added at once or continuously during a reaction. The reaction can be carried out by a batch or continuous reaction.

Optically active 3-substituted glutaric acid monoamide generated as a result of the reaction can be isolated and purified by a standard method. For instance, a reaction solution containing optically active 3-substituted glutaric acid monoamide generated as a result of a hydrolysis reaction is extracted using an organic solvent such as ethyl acetate or toluene, and the organic solvent is removed under reduced pressure, followed by treatment such as distillation, recrystallization, or chromatography. Thus, isolation and purification can be carried out. Alternatively, a filtrate obtained by removing microbial cells from a reaction solution is subjected to neutralization/crystallization with the use of sulfuric acid or the like. The resulting precipitate of interest is filtered for separation. Thus, isolation and purification can be carried out.

## Examples

The present invention is hereafter described in greater detail with reference to the following examples, although the present invention is not limited thereto.

### (Example 1) Stereoselective hydrolysis of 3-propyl glutaric acid diamide

Each of the microorganisms listed in tables 1 and 2 was separately seeded on a sterilized medium (glycerol: 1.0%; peptone: 0.5%; malt extract: 0.3%; yeast extract: 0.3%; ε-caprolactam: 0.1%; pH 7.0) (8 mL) in a test tube, followed by shaking culture at 30°C for 72 hours. After the end of culture, microbial cells were harvested via centrifugation and suspended in 100 mM phosphate buffer (2 mL) (pH 7.0).

The obtained cell suspensions (1 mL each) were separately mixed with a substrate (3-propyl glutaric acid diamide; 2 mg) synthesized by the method described in Reference Example 1, and each of the mixtures was shaken at 30°C for 24 hours. After the end of reaction, the resulting solid matter was removed via centrifugation. The substrate and the product in the reaction solution were analyzed by high-performance liquid chromatography so as to determine the conversion rate (%).

Further, the product in the reaction solution was derivatized using phenacyl bromide. The thus obtained derivative was analyzed by high-performance liquid chromatography so as to determine the optical purity (%ee).

Tables 1 and 2 show the results.

[Table 1]

| Microorganism | Conversion rate (%) | Optical purity (%ee) | Absolute configuration |
|---|---|---|---|
| *Achromobacter sp.* | 5.9 | 45.1 | S |
| *Arthrobacter* sp. | 7.9 | 78.5 | S |
| *Delftia acidovorans* | 24.9 | 55.5 | S |
| *Delftia acidovorans* NBRC13582 | 15.0 | 67.2 | S |
| *Stenotrophomonas sp.* | 8.1 | 62.4 | S |

[Table 2]

| Microorganism | Conversion rate (%) | Optical purity (%ee) | Absolute configuration |
|---|---|---|---|
| *Atealigenes* sp. IFO14130 | 19.8 | 37.3 | R |
| *Acinetobacter* sp. | 93.6 | 70.2 | R |
| *Acinetobacter* sp. | 76.8 | 76.1 | R |
| *Nocardia globerula* | 57.1 | 88.6 | R |
| *Rhodococcus erythropolis* IFO12538 | 51.3 | 72.0 | R |
| *Rhodococcus erythropolis* IFO12539 | 89.5 | 76.8 | R |
| *Rhodococcus erythropolis* IAM1452 | 23.7 | 67.7 | R |
| *Rhodococcus erythropolis* IAM1474 | 82.8 | 77.9 | R |
| *Rhodococcus erythropolis* IAM12122 | 90.6 | 82.0 | R |
| *Rhodococcus rhodochrous* NBRC15564 | 61.6 | 81.5 | R |

$$\text{Conversion rate (\%)} = \text{Product amount}/(\text{Substrate amount} + \text{Product amount}) \times 100$$

$$\text{Optical purity (\%ee)} = (A - B)/(A + B) \times 100$$

(Each of A and B represents the amount of a relevant enantiomer (A > B).)

<High-performance liquid chromatography analysis conditions>

[Conversion rate analysis]

Column: 5C18-ARII (4.6 min (ϕ) x 250 mm; Nacalai Tesque)

Eluent: 20 mM phosphoric acid aqueous solution (pH 2.5) : acetonitrile = 8:2

Flow rate: 1.0 mL/minute; column temperature: 30°C; measurement wavelength: 210 nm

[Optical purity analysis]

Column: CHIRALPACK AD-RH (4.6 mm (ϕ) x 150 mm; Daicel Corporation)
Eluent: 20 mM phosphoric acid aqueous solution (pH 2.5) : acetonitrile = 1:1
Flow rate: 0.5 mL/minute; column temperature: room temperature;
measurement wavelength: 210 nm

(Example 2) Stereoselective hydrolysis of 3-(4-chlorophenyl) glutaric acid diamide

Each of the microorganisms listed in tables 3 and 4 was separately seeded on a sterilized medium (glycerol: 1.0%; peptone: 0.5%; malt extract: 0.3%; yeast extract: 0.3%; ε-caprolactam: 0.1%; pH 7.0) (8 mL) in a test tube, followed by shaking culture at 30°C for 72 hours. After the end of culture, microbial cells were harvested via centrifugation and suspended in 100 mM phosphate buffer (2 mL) (pH 7.0).
The obtained cell suspensions (1 mL each) were separately mixed with a substrate (3-(4-chlorophenyl) glutaric acid diamide; 2 mg) synthesized by the method described in Reference Example 2 and each of the mixtures was shaken at 30°C for 24 hours. After the end of reaction, the resulting solid matter was removed via centrifugation. The substrate and a product in the reaction solution were analyzed by high-performance liquid chromatography so as to determine the conversion rate (%) and the optical purity (%ee).
Tables 3 and 4 show the results.
[Table 3]

| Microorganism | Conversion rate (%) | Optical purity (%ee) | Absolute configuration |
|---|---|---|---|
| *Achromobacter* sp. | 20.2 | 97.4 | S |
| *Achromobacter xylosoxidans* subsp. *enitrificans* IFO15125 | 22.0 | 94.4 | S |
| *Achromobacter xylosoxidans* subsp, *denitrificans* IFO 12669 | 21.0 | 96.2 | S |
| *Alcaligenes xylosoxidans* subsp. *denitrificans* | 50.1 | 99.0 | S |
| *Arthrobacter* sp. | 38.3 | 99.0 | S |
| *Acinetobacter* sp. | 19.0 | 8.4 | S |
| *Cellulomonas fimi* IFO15513 | 72.2 | 6.0 | S |
| *Delftia acidovorans* NBRC13582 | 96.5 | 99.0 | S |
| *Delftia acidovorans* NBRC149950 | 80.0 | 99.0 | S |
| *Pseudomonas* sp. | 14.0 | 94.7 | S |
| *Stenotrophomonas* sp. | 86.8 | 99.0 | S |

[Table 4]

| Microorganism | Conversion rate (%) | Optical purity (%ee) | Absolute configuration |
|---|---|---|---|
| *Comamonas* sp. KNK3-7 (NITE BP-963) | 43.0 | 98.0 | R |
| *Rhodococcus erythropolis* IAM1440 | 53.1 | 11.1 | R |
| *Rhodococcus erythropolis* IAM1474 | 9.8 | 6.1 | R |
| *Rhodococcus erythropolis* IAM12122 | 11.3 | 9.0 | R |
| *Rhodococcus erythropolis* | 96.4 | 47.1 | R |

(continued)

| Microorganism | Conversion rate (%) | Optical purity (%ee) | Absolute configuration |
|---|---|---|---|
| *Rhodococcus erythropolis* | 40.1 | 18.4 | R |
| *Pseudomonas* sp. KNK24-9 (NITE BP-962) | 14.0 | 97.0 | R |

$$\text{Conversion rate (\%)} = \text{Product amount}/(\text{Substrate amount} + \text{Product amount}) \times 100$$

$$\text{Optical purity (\%ee)} = (A - B)/(A + B) \times 100$$

(Each of A and B represents the amount of a relevant enantiomer (A > B).)

<High-performance liquid chromatography analysis conditions>

[Conversion rate analysis]

Column: 5C18-ARII (4.6 mm (ϕ) x 250 mm; Nacalai Tesque)

Eluent: 20 mM phosphoric acid aqueous solution (pH 2.5) : acetonitrile = 7:3

Flow rate: 1.0 mL/minute; column temperature: 30°C; measurement

wavelength: 210 nm

[Optical purity analysis]

Column: SUMICHIRAL OA-7000 (4.6 mm (ϕ) x 250 mm; Sumika Chemical Analysis Service, Ltd)
Eluent: 20 mM phosphoric acid aqueous solution (pH 2.5) : acetonitrile = 8:2
Flow rate: 0.5 ml/minute; column temperature: room temperature;
measurement wavelength: 210 nm

(Reference Example 1) Synthesis of 3-(4-chlorophenyl) glutaric acid diamide

3-(4-chlorophenyl) glutaric acid (10.0 g; 41.2 mmol) was dissolved in methanol (60 mL). Thionyl chloride (12.3 g; 103 mmol) was added dropwise thereto. After the end of dropwise addition, the resultant was stirred for 2 hours, followed by distillation of the solvent at reduced pressure. Thus, a white solid of 3-(4-chlorophenyl) glutaric acid dimethyl (11.1 g; 41.2 mmol) was obtained.
The solid of 3-(4-chlorophenyl) glutaric acid dimethyl (8.0 g; 30.0 mmol) was placed in a pressure-proof container. An ammonia/methanol solution (wet weight: 80 g; 800 mmol) was added thereto. Then, the container was heated in a hermetically sealed system at 80°C.
Stirring was conducted for 3 days. Thereafter, the content of the container was subjected to crude concentration. The obtained solid precipitate was filtered for separation. The filtered solid matter was dried at reduced pressure. Thus, a white solid of 3-(4-chlorophenyl) glutaric acid diamide (6.5 g; 27.0 mmol) was obtained.

(Reference Example 2) Synthesis of 3-propyl glutaric acid diamide

3-propyl glutaric acid (8.0 g, 45.9 mmol) was dissolved in methanol (50 mL). Thionyl chloride (13.7 g; 115 mmol) was added dropwise thereto. After the end of dropwise addition, the resultant was stirred for 2 hours, followed by distillation of the solvent at reduced pressure. Thus, an oily matter of 3-propyl glutaric acid dimethyl (9.3 g; 45.9 mmol) was obtained.
The oily matter of 3-propyl glutaric acid dimethyl (9.0 g; 44.5 mmol) was placed in a pressure-proof container. An ammonia/methanol solution (wet weight: 90 g; 900 mmol) was added thereto. Then, the container was heated in a hermetically sealed system at 80°C.
Stirring was conducted for 5 days. Thereafter, the content of the container was subjected to crude concentration. The obtained solid precipitate was filtered for separation. The filtered solid matter was dried at reduced pressure. Thus, a solid of 3-propyl glutaric acid diamide (2.3 g; 13.4 mmol) was obtained.

## Claims

1. A method for producing optically active 3-substituted glutaric acid mono amide of the following formula (2), comprising allowing an enzyme source having activity of asymmetrically hydrolyzing a 3-substituted glutaric acid diamide of the following formula (1) to act on the compound of (1):

Formula (1)

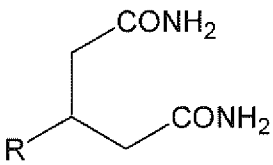

(1)

(where R represents a $C_1$-$C_8$ alkyl group that may optionally have a substituent, a $C_2$-$C_8$ alkenyl group that may optionally have a substituent, a $C_2$-$C_8$ alkynyl group that may optionally have a substituent, a $C_4$-$C_{20}$ aryl group that may optionally have a substituent, or a $C_5$-$C_{20}$ aralkyl group that may optionally have a substituent);

Formula (2)

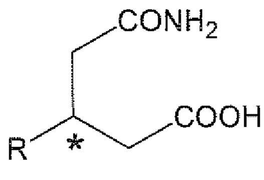

(2)

(where "*" represents an asymmetric carbon atom and R has the same definition as that above).

2. The production method according to claim 1, wherein R represents a $C_1$-$C_8$ alkyl group that may optionally have a substituent or a $C_4$-$C_{20}$ aryl group that may optionally have a substituent.

3. The production method according to claim 1, wherein R represents a propyl group, an isobutyl group, a phenyl group, or a 4-chlorophenyl group.

4. The production method according to any one of claims 1 to 3, wherein the enzyme source comprises microbial cells

of at least one microorganism selected from the group consisting of the following and/or a processed product thereof: members of the genera *Achromobacter, Alcaligenes, Arthrobacter, Acinetobacter, Cellulomonas, Comamonas, Delftia, Nocardia, Pseudomonas, Rhodococcus,* and *Stenotrophomonas.*

**5.** The production method according to any one of claims 1 to 4, wherein the enzyme source comprises microbial cells of at least one microorganism selected from the group consisting of the following and/or a processed product thereof: the genera *Achromobacter* sp.; *Achromobacter xylosoxidans* subsp. *denitrificans*; *Alcaligenes* sp.; *Alcaligenes xylosoxidans* subsp. *denitrificans*; *Arthrobacter* sp.; *Acinetobacter* sp.; *Cellulomonas fimi*; *Comamonas* sp.; *Delftia acidovorans*; *Nocardia globerula*; *Pseudomonas* sp.; *Rhodococcus erythropolis*; *Rhodococcus rhodochrous*; and *Stenotrophomonas* sp.

**6.** The production method according to claim 4, wherein a 3-substituted glutaric acid monoamide compound for which the absolute configuration is the S-configuration is produced using an enzyme source comprising microbial cells of at least one microorganism selected from the group consisting of the following and/or a processed product thereof: members of the genera *Achromobacter, Alcaligenes, Arthrobacter, Acinetobacter, Cellulomonas, Delftia, Pseudonionas,* and *Stenotrophomonas.*

**7.** The production method according to claim 6, wherein a 3-substituted glutaric acid monoamide compound for which the absolute configuration is the S-configuration is produced using an enzyme source comprising microbial cells of at least one microorganism selected from the group consisting of the following and/or a processed product thereof: *Achromobacter* sp.; *Achromobacter xylosoxidans* subsp. *denitrificans; Alcaligenes xylosoxidans* subsp. *denitrificans; Arthrobacter* sp.; *Acinetobacter* sp.; *Cellulomonas fimi; Delftia acidovorans; Pseudomonas* sp.; and *Stenotrophomonas* sp.

**8.** The production method according to claim 4, wherein 3-substituted glutaric acid monoamide for which the absolute configuration is the R-configuration is produced using an enzyme source comprising microbial cells of at least one microorganism selected from the group consisting of the following and/or a processed product thereof: members of the genera *Alcaligenes, Acinetobacter, Comamonas, Nocardia, Rhodococcus,* and *Pseudonaonas.*

**9.** The production method according to claim 8, wherein 3-substituted glutaric acid monoamide for which the absolute configuration is the R-configuration is produced using an enzyme source comprising microbial cells of at least one microorganism selected from the group consisting of the following and/or a processed product thereof: *Alcaligenes* sp., *Acinetobacter* sp., *Comamonas* sp., *Nocardia globerula, Rhodococcus erythropolis, Rhodococcus rhodochrous,* and *Pseudomonas* sp.

**INTERNATIONAL SEARCH REPORT**

International application No.
PCT/JP2010/004736

A. CLASSIFICATION OF SUBJECT MATTER
*C12P41/00*(2006.01)i

According to International Patent Classification (IPC) or to both national classification and IPC

B. FIELDS SEARCHED

Minimum documentation searched (classification system followed by classification symbols)
C12P41/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 97/022578 A1 (Farmarc Nederland B.V.), 26 June 1997 (26.06.1997), & US 6051734 A & EP 874804 A | 1-9 |
| A | FELLUGA F. et al., A short and convenient chemoenzymatic synthesis of both enantiomers of 3-phenylGABA and 3-(4-chlorophenyl)GABA (Baclofen), Tetrahedron Asym., 2005, vol.16, no.7, p.1341-1345 | 1-9 |
| A | LEVADOUX W.et al., Microbial resolution of baclofen by a new isolate of Streptomyces halstedii, J. Biosci. Bioeng., 2002, vol.93, no.6, p.557-562 | 1-9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 October, 2010 (01.10.10) | 12 October, 2010 (12.10.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 97225 A

### Non-patent literature cited in the description

- *Journal of the Chemical Society, Perkin Transactions,* 1997, vol. 2 (4), 763-768
- *Can. J. Chem.,* 1994, vol. 72, 2312